(19) **Europäisches Patentamt** / **European Patent Office** / **Office européen des brevets**

(11) **EP 3 777 825 A1**

(12) # EUROPEAN PATENT APPLICATION

(43) Date of publication:
17.02.2021 Bulletin 2021/07

(51) Int Cl.:
*A61K 8/73* (2006.01)  *A61Q 19/00* (2006.01)

(21) Application number: 20188071.3

(22) Date of filing: 28.07.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 08.08.2019 KR 20190096784

(71) Applicant: **Amorepacific Corporation**
**Seoul 04386, (KR)**

(72) Inventors:
• JANG, Jiwook
17074 Gyeonggi-do (KR)
• CHO, Sungyeon
17074 Gyeonggi-do (KR)
• CHOI, Kyungho
17074 Gyeonggi-do (KR)

(74) Representative: **Agasse, Stéphane et al**
**Cabinet GERMAIN & MAUREAU**
**B.P. 6153**
**69466 Lyon Cedex 06 (FR)**

(54) **COMPOSITION OF FREEZE-DRIED FORMULATION, COSMETIC KIT FOR EXTERNAL USE ON SKIN INCLUDING THE SAME, AND ITS USE**

(57)  Disclosed is a composition of freeze-dried formulation which is mixed with a medium composition to be applied to the skin. Based on the total weight of the composition of freeze-dried formulation and the medium composition, freeze-dried hyaluronic acid is present in an amount of 0.5 dry wt% or more. The freeze-dried hyaluronic acid includes crosslinked hyaluronic acid and low-molecular weight hyaluronic acid at a weight ratio of 1:5 to 3:1. Thus, the composition allows incorporation of a high concentration of hyaluronic acid capable of retaining a moisturizing protective film, while supplementing skin constitutional ingredients, provides a high moisturizing effect without slipperiness and stickiness, has a dried form and requires no preservative, or the like, and can stabilize an unstable active ingredient.

[Fig. 1]

Example 1 · Example 2 · Comparative Example 1 · Comparative Example 2 · Comparative Example 3

EP 3 777 825 A1

**Description**

[Technical Field]

The present disclosure relates to a composition of freeze-dried formulation, a cosmetic kit for external use on skin including the same, and its use.

[Background Art]

Since freeze-dried cosmetics have no water in their formulations, they are advantageous to supporting active ingredients susceptible to water and solvents. In addition, freeze-dried cosmetics require no preservative treatment, and thus can be used as preservative-free formulations. Such a freeze-dried cosmetic formulation should be used after it is dissolved in a solvent, such as water or essence, and shows a significantly low dissolution rate or significantly low viscosity depending on its formulation to cause inconvenience of use in some cases.

Meanwhile, hyaluronic acid is a substance forming the body and found in the body fluid, bovine eyeball, cockscomb, cushioning tissues of animals, placenta, or the like, and is present at a high concentration particularly in the connective tissues and skin. In addition, hyaluronic acid is capable of containing water in an amount corresponding to approximately 1000 times of its weight. Thus, it has been used widely as a moisturizing agent for cosmetics to prevent the skin from drying. In the field of skin cosmetics, hyaluronic acid has been used as a dermal filler inserted to a specific site to extend soft tissues, thereby improving wrinkles or correcting face lines.

However, there is a problem in that it is difficult to use hyaluronic acid at a high concentration due to the slipperiness and stickiness unique to hyaluronic acid. In a freeze-dried cosmetic formulation, there is also a problem of severe slipperiness and stickiness, when using hyaluronic acid.

**[Disclosure]**

[Technical Problem]

A technical problem to be solved by the present disclosure is to provide a freeze-dried hyaluronic acid composition for external use on skin which requires no preservative and can stabilize an unstable active ingredient, while providing a high moisturizing effect without slipperiness and stickiness.

[Technical Solution]

In one general aspect, there is provided a composition of freeze-dried formulation which is mixed with a medium composition to be applied to the skin and includes freeze-dried hyaluronic acid, wherein the freeze-dried hyaluronic acid includes crosslinked hyaluronic acid and low-molecular weight hyaluronic acid, the freeze-dried hyaluronic acid is present in an amount of 0.5 dry wt% or more based on a total weight of the composition of freeze-dried formulation and the medium composition, when the composition of freeze-dried formulation is mixed with the medium composition to be applied to the skin, and a weight ratio of the crosslinked hyaluronic acid to the low-molecular weight hyaluronic acid is 1:5 to 3:1.

In another general aspect, there is provided a cosmetic kit for external use on skin which includes: a first agent which is the above-mentioned composition of freeze-dried formulation; and a second agent which is a medium composition mixed with the first agent to be applied to the skin, wherein the first agent and the second agent are mixed with each other to form a gel composition for external use on skin, and the gel composition for external use on skin includes freeze-dried hyaluronic acid in an amount of 0.5 dry wt% or more based on the total weight of the gel composition for external use on skin.

[Advantageous Effects]

According to an embodiment of the present disclosure, the freeze-dried hyaluronic acid composition for external use on skin includes crosslinked hyaluronic acid at a high concentration, and provides low stickiness and a fresh feeling of use, while showing improved spreadability and applicability and an excellent skin moisturizing effect.

According to another embodiment of the present disclosure, the freeze-dried hyaluronic acid composition for external use on skin requires no preservative by virtue of its freeze-dried formulation, and can stabilize an unstable active ingredient.

[Brief Description of Drawings]

FIG. 1 illustrates the results of observation of the freeze-dried hyaluronic acid compositions for external use on skin according to Examples 1 and 2 and Comparative Examples 1-3, after freeze-drying.
FIG. 2 illustrates the results of observation of solubility for each of the freeze-dried hyaluronic acid compositions for external use on skin according to Examples 1 and 2 and Comparative Examples 1-3.

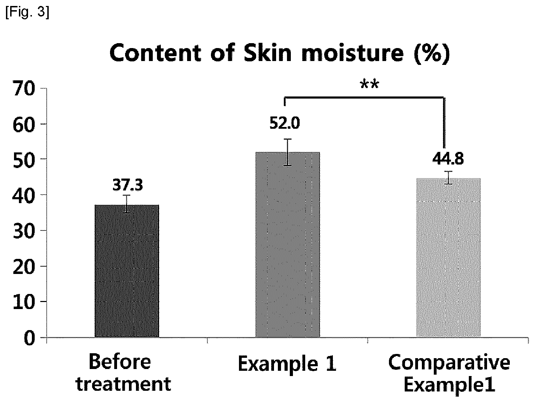

FIG. 3 illustrates the results of determination of skin moisturizing effects for the freeze-dried hyaluronic acid compositions for external use on skin according to Example 1 and Comparative Example 1, when applying them to the skin.

[Best Mode]

Throughout the specification, the expression 'a part 「comprises」 an element' does not preclude the presence of any additional elements but means that the part may further include the other elements, unless otherwise stated.
As used herein, 'composition for external use on skin' is a generic term covering any compositions applied from the outside of the skin, and includes various formulations of cosmetic products and medicines.
Hereinafter, the present disclosure will be explained in detail.
In one aspect of the present disclosure, there is provided a composition of freeze-dried formulation which is mixed with a medium composition to be applied to the skin and includes freeze-dried hyaluronic acid, wherein the freeze-dried hyaluronic acid includes crosslinked hyaluronic acid and low-molecular weight hyaluronic acid, the freeze-dried hyaluronic acid is present in an amount of 0.5 dry wt% or more based on the total weight of the composition of freeze-dried formulation and the medium composition, when the composition of freeze-dried formulation is mixed with the medium composition to be applied to the skin, and the weight ratio of the crosslinked hyaluronic acid to the low-molecular weight hyaluronic acid is 1:5 to 3:1.
According to the related art, hyaluronic acid is an ingredient effective for moisturization but has a disadvantage in that it is severely slippery and sticky and thus is hardly used at a high concentration in a cosmetic formulation. In a freeze-dried cosmetic formulation, there is also a problem of severe slipperiness and stickiness when using hyaluronic acid. In addition, low-molecular weight hyaluronic acid permeates the skin to provide an effect upon skin collagen and HA synthesis, and crosslinked (polymeric) HA is coated on the skin and shows an excellent moisturizing effect. Under these circumstances, there has been a need for developing a composition having both of the above-mentioned advantages.
To overcome this, the present inventors have focused on the fact that crosslinked hyaluronic acid provides improvement in terms of stickiness and slipperiness and shows a higher skin moisturizing effect as compared to low-molecular weight (non-crosslinked) hyaluronic acid. Therefore, we have found a composition of freeze-dried formulation, which further includes low-molecular weight hyaluronic acid to allow incorporation of a high concentration of hyaluronic acid capable of retaining a moisturizing protective film, while supplementing skin constitutional ingredients, and provides a high moisturizing effect without slipperiness and stickiness.
In addition, the composition of freeze-dried formulation according to an embodiment of the present disclosure has a dried form and requires no preservative, or the like, and can stabilize an unstable active ingredient. Therefore, after the composition is prepared in a dried form including an active ingredient, such as vitamin, it may be mixed with various medium compositions to be used as a gel-like composition for external use on skin, and thus may be utilized as various functional compositions for external use on skin.
The crosslinked hyaluronic acid may be prepared by crosslinking hyaluronic acid in a basic solution by using an epoxide-based crosslinking agent. Herein, since the crosslinked hyaluronic acid may cause irritation in the human body due to ether bonding, it may be washed.
Then, the crosslinked hyaluronic acid and low-molecular weight hyaluronic acid may be mixed with a solvent and an active ingredient, and then the resultant mixture may be freeze-dried to obtain a finished composition of freeze-dried formulation. For example, the solvent may be a water-soluble solvent, such as purified water, a lower alcohol, including ethanol, or the like, preferably.
The freeze-drying may be carried out by using a freeze-dryer at a temperature of -10°C or lower, or -20°C or lower, and the freeze-dryer is not particularly limited, as long as it is one used generally in the art.
According to an embodiment, the term 'crosslinking' may refer to ether bonding between the OH group of hyaluronic acid and the epoxide-based crosslinking agent.
According to an embodiment, the crosslinked hyaluronic acid may be crosslinked with an epoxide-based crosslinking agent.
According to an embodiment, the epoxide-based crosslinking agent may be at least one selected from the group consisting of 1,4-butanediol diglycidyl ether (BDDE), ethylene glycol diglycidyl ether (EGDGE), 1-(2,3-epoxypropyl)-2,3-epoxycyclohexane, 1,2-ethanediol diglycidyl ether, 1,6-hexanediol diglycidyl ether, propylene glycol diglycidyl ether, polypropylene glycol diglycidyl ether, and diglycerol polyglycidyl ether. Preferably, the epoxide-based crosslinking agent may be 1,4-butanediol diglycidyl ether (BDDE).

The content of the crosslinked hyaluronic acid and low-molecular weight hyaluronic acid may be 0.5 dry wt% or more, 0.6 dry wt% or more, 0.7 dry wt% or more, 0.8 dry wt% or more, 0.9 dry wt% or more, or 1 dry wt% or more, and 2 dry wt% or less, 1.9 dry wt% or less, 1.8 dry wt% or less, 1.7 dry wt% or less, or 1.6 dry wt% or less, based on the total weight of the freeze-dried hyaluronic acid cosmetic composition.

When the content is less than 0.5 dry wt%, the composition shows degradation of a moisturizing effect, when using the composition after the freeze-dried formulation is dissolved in a solvent, or the freeze-dried formulation cannot form a gel-like shape. When the content is larger than 2 dry wt%, the composition shows poor applicability, and thus a stiff feeling of use and dirt-rubbing may be generated upon skin application.

According to an embodiment of the present disclosure, the weight ratio of the crosslinked hyaluronic acid to low-molecular weight hyaluronic acid may be 1:5 to 3:1, 1:4 to 2:1, or 1:3 to 1:1. When the weight ratio is less than 1:5, the amount of crosslinked hyaluronic acid is excessively small to cause degradation of a moisturizing effect and a sticky inconvenient feeling. When the weight ratio is larger than 3:1, the amount of crosslinked hyaluronic acid is excessively large, and thus the composition cannot retain its formulation well, cannot be dissolved well, when being applied to essence, or the like, or may show a stiff feeling of use and dirt-rubbing.

According to an embodiment of the present disclosure, the crosslinked hyaluronic acid may have a weight average molecular weight of 10,000,000 Da or more, and the low-molecular weight hyaluronic acid may have a weight average molecular weight of 5,000 Da or less.

For example, the crosslinked hyaluronic acid may have a weight average molecular weight of 15,000,000 Da or more, 20,000,000 Da or more, or 25,000,000 Da or more, and 50,000,000 Da or less, and the low-molecular weight hyaluronic acid may have a weight average molecular weight of 5,000 Da or less, 4,000 Da or less, or 3,000 Da or less, and 1,000 Da or more.

When the crosslinked hyaluronic acid has a weight average molecular weight less than 10,000,000 Da, moisturizing effect may be degraded. When the low-molecular weight hyaluronic acid has a weight average molecular weight larger than 5,000 Da, there may be a difficulty in skin permeation.

According to an embodiment of the present disclosure, the crosslinked hyaluronic acid may have a particle size less than 200 μm. The particle size is determined as the maximum length, which is the largest length of crosslinked hyaluronic acid particles. As used herein, the particle size range of crosslinked hyaluronic acid means that at least 90%, at least 95%, or at least 99% of the crosslinked hyaluronic acid particles present in the composition fall within the above-defined range.

For example, the crosslinked hyaluronic acid may have a particle size of 150 μm or less, 100 μm or less, or 50 μm or less, and 5 μm or more, 10 μm or more, or 15 μm or more. When the particle size is larger than 200 μm, dirt-rubbing may occur, and the freeze-dried formulation cannot absorb water completely so that it may not be converted into a gel-like shape.

According to an embodiment of the present disclosure, the composition of freeze-dried formulation may further include, as an active ingredient, at least one selected from the group consisting of water-soluble vitamins, oil-soluble vitamins, vitamin derivatives, polymeric peptides, polysaccharides, spingolipids and seaweed extract.

According to an embodiment of the present disclosure, the composition of freeze-dried formulation may be used for skin moisturization.

According to an embodiment, the composition of freeze-dried formulation may be a cosmetic composition, and the outer shape of the cosmetic composition includes a cosmetically or dermatologically acceptable medium or base. The composition may include any formulations suitable for local application. For example, the composition may be provided in the form of a solution, gel, solid, dry slurry product, emulsion prepared by dispersing an oil phase in an aqueous phase, suspension, microemulsion, microcapsules, microgranules or ionic (liposome) and non-ionic sachet dispersant, or provided as cream, skin, lotion, powder, ointment, spray or conceal stick. Such formulations may be obtained by the method generally known to those skilled in the art. In addition, the cosmetic composition may be used in the form of foam or an aerosol composition further including a compressed propellant.

The cosmetic composition according to an embodiment of the present disclosure is not limited to any particular formulation. For example, the cosmetic composition may be formulated into cosmetic products, such as ampoule, cream, skin softener, skin astringent, skin nutrient, nutrient cream, massage cream, essence, eye cream, eye essence, cleansing cream, cleansing foam, cleansing water, cleansing tissue containing the cosmetic composition, pack, powder, body lotion, body cream, body oil and body essence.

When the formulation is paste, cream or gel, carrier ingredients that may be used include animal fibers, vegetable fibers, wax, paraffin, starch, tragacanth, cellulose derivatives, polyethylene glycol, silicone, bentonite, silica, talc or zinc oxide.

When the formulation is powder or spray, carrier ingredients that may be used include lactose, talc, silica, aluminum hydroxide, calcium silicate or polyamide powder. Particularly, in the case of spray, it may further include a propellent, such as chlorofluorohydrocarbon, propane/butane or dimethyl ether.

When the formulation is a solution or emulsion, carrier ingredients that may be used include a solvent, solvating agent or emulsifying agent, such as water, ethanol, isopropanol, ethyl carbonate, ethyl acetate, benzyl alcohol, benzyl benzoate, propylene glycol, 1,3-butylglycol oil, glycerol fatty acid ester, polyethylene glycol or sorbitan fatty acid ester.

When the formulation is a suspension, carrier ingredients that may be used include a liquid diluent, such as water, ethanol or propylene glycol, a suspending agent, such as ethoxylated isostearyl alcohol, polyoxyethylene sorbitol ester or polyoxyethylene sorbitan ester, microcrystalline cellulose, aluminum metahydroxide, bentonite, agar, tragacanth, or the like.

When the formulation is a surfactant-containing cleanser, carrier ingredients that may be used include aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamine, vegetable oil, lanoline derivative, ethoxylated glycerol fatty acid ester, or the like.

The cosmetic composition according to an embodiment of the present disclosure may further include functional additives and include other ingredients used currently in a cosmetic composition. The functional additive may include an ingredient selected from the group consisting of water soluble vitamins, oil soluble vitamins, polymer peptides, polymer polysaccharides, spingolipids and seaweed extract.

In addition to the above-mentioned ingredients, the cosmetic composition according to an embodiment of the present disclosure may further include an oil and fat ingredient, moisturizing agent, emollient, surfactant, organic and inorganic pigments, organic powder, preservative, sterilizing agent, antioxidant, plant extract, pH modifier, alcohol, colorant, fragrance, blood flow-stimulating agent, coolant, anti-perspirant, purified water, or the like.

According to an embodiment, the composition of freeze-dried formulation may be a pharmaceutical composition. When applying the composition of freeze-dried formulation to medicines, an inorganic or organic carrier used currently for the active ingredient used in the present disclosure may be added so that the composition may be formulated into a semi-solid or liquid formulation for parenteral administration. The pharmaceutical composition according to the present disclosure may be administered through a parenteral, rectal, local, transdermal, intravenous, intramuscular, intraperitoneal, subcutaneous route, or the like.

The composition according to the present disclosure may be easily formulated with an active ingredient according to the method generally known to those skilled in the art, wherein a surfactant, vehicle, colorant, fragrance, preservative, stabilizer, buffer, suspending agent, or other conventional adjuvants may be used suitably.

The effective administration dose of the pharmaceutical composition according to an embodiment of the present disclosure may be varied depending on age, sex, body weight, pathological condition and severity of a subject to be administered, administration route or judgement of a prescriber. Determination of a suitable dose may be made by those skilled in the art based on the above-mentioned factors. For example, the pharmaceutical composition may be administered in a daily dose of 0.1-100 mg/kg/day, particularly 5-50 mg/kg/day, but is not limited thereto.

In another aspect of the present disclosure, there is provided a cosmetic kit for external use on skin which includes: a first agent which is the above-mentioned composition of freeze-dried formulation; and a second agent which is a medium composition mixed with the first agent to be applied to the skin, wherein the first agent and the second agent are mixed with each other to form a gel composition for external use on skin, and the gel composition for external use on skin includes freeze-dried hyaluronic acid in an amount of 0.5 dry wt% or more based on the total weight of the gel composition for external use on skin.

According to an embodiment of the present disclosure, the second agent may be at least one selected from the group consisting of ampoule, cream, skin softener, skin astringent, skin nutrient, nutrient cream, massage cream, essence, eye cream, eye essence, cleansing cream and cleansing water. Preferably, the second agent may be at least one selected from the group consisting of ampoule, skin softener, skin astringent, skin nutrient, eye essence and cleansing water.

The composition of freeze-dried formulation according to an embodiment of the present disclosure rapidly absorbs the solvent in the medium composition to form a gel composition for external use on skin. Therefore, the composition of freeze-dried formulation can be used as a gel composition for external use on skin, which has an excellent moisturizing effect and includes various active ingredient, in a simple manner.

According to an embodiment, the gel composition for external use on skin may have a tanδ value of 0.5-1.5, as determined according to the following Formula 1:

[Formula 1]

$$Tan\delta = G''/G'$$

wherein G" is the loss modulus of the gel composition for external use on skin, and G' is the storage modulus of the gel composition for external use on skin.

Herein, each of G" and G' is expressed in the unit of Pa, and may be determined by using a rheometer with a 20 mm plate at 25°C under the conditions of a frequency range of 0.1-10 Hz, shear stress of 1 Pa and a gap of 1 mm, and reading G' and G" values at 1Hz.

For example, the tanδ value may be 0.6 or more, 0.7 or more, or 0.8 or more, and 1.4 or less, 1.3 or less, or 1.2 or less. When the tanδ value is less than 0.5, the composition is nearly a hard solid and shows poor applicability. When the tanδ value is larger than 1.5, it is nearly liquid, not gel, and thus is not suitable for formation of a moisturizing film.

In still another aspect of the present disclosure, there is provided a method for improving skin moisturizing ability by applying a composition of freeze-dried formulation onto the skin, wherein the composition of freeze-dried formulation is mixed with a medium composition and applied to the skin, the composition of freeze-dried formulation includes freeze-dried hyaluronic acid, the freeze-dried hyaluronic acid includes crosslinked hyaluronic acid and low-molecular weight hyaluronic acid, the freeze-dried hyaluronic acid is present in an amount of 0.5 dry wt% or more based on the total weight of the composition of freeze-dried formulation and the medium composition, when the composition of freeze-dried formulation is mixed with the medium composition and applied to the skin, and the weight ratio of the crosslinked hyaluronic acid to the low-molecular weight hyaluronic acid is 1:5 to 3:1.

In still another aspect of the present disclosure, there is provided use of a composition of freeze-dried formulation for the preparation of a skin moisturizing composition for external use on skin, wherein the composition of freeze-dried formulation is mixed with a medium composition to be applied to the skin, the composition of freeze-dried formulation includes freeze-dried hyaluronic acid, the freeze-dried hyaluronic acid includes crosslinked hyaluronic acid and low-molecular weight hyaluronic acid, the freeze-dried hyaluronic acid is present in an amount of 0.5 dry wt% or more based on the total weight of the composition of freeze-dried formulation and the medium composition, when the composition of freeze-dried formulation is mixed with the medium composition to be applied to the skin, and the weight ratio of the crosslinked hyaluronic acid to the low-molecular weight hyaluronic acid is 1:5 to 3:1.

In yet another aspect of the present disclosure, there is provided a composition of freeze-dried formulation for use in skin moisturization, wherein the composition of freeze-dried formulation is mixed with a medium composition to be applied to the skin, the composition of freeze-dried formulation includes freeze-dried hyaluronic acid, the freeze-dried hyaluronic acid includes crosslinked hyaluronic acid and low-molecular weight hyaluronic acid, the freeze-dried hyaluronic acid is present in an amount of 0.5 dry wt% or more based on the total weight of the composition of freeze-dried formulation and the medium composition, when the composition of freeze-dried formulation is mixed with the medium composition to be applied to the skin, and the weight ratio of the crosslinked hyaluronic acid to the low-molecular weight hyaluronic acid is 1:5 to 3:1.

Hereinafter, the present disclosure will be explained in more detail with reference to examples. However, the following examples are for illustrative purposes only. In addition, it will be apparent to those skilled in the art that the scope of the present disclosure is not limited to the following examples.

**Examples**

The composition of freeze-dried formulation according to each of Examples 1 and 2 and Comparative Examples 1-3 was prepared as shown in the following Table 1.

[Table 1]

|  | Ex. 1 | Ex. 2 | Comp. Ex. 1 | Comp. Ex. 2 | Comp. Ex. 3 |
|---|---|---|---|---|---|
| 20 μm crosslinked hyaluronic acid gel | 5 |  |  |  |  |
| 200 μm crosslinked hyaluronic acid gel |  | 5 |  |  |  |
| Polymeric hyaluronic acid |  |  |  | 5 |  |
| Low-molecular weight hyaluronic acid | 10 | 10 | 15 | 10 | 10 |
| Carboxymethyl |  |  |  |  | 5 |
| cellulose |  |  |  |  |  |
| Vitamin C | 5 | 5 | 5 | 5 | 5 |
| Purified water | 980 | 980 | 980 | 980 | 980 |

[Example 1]

<Preparation of Crosslinked Hyaluronic Acid>

In this example, 20 wt% of sodium hyaluronate was dissolved in 0.25N NaOH and 0.1 wt% of BDDE was added thereto to carry out crosslinking at 37°C for 24 hours. After crosslinking, the resultant gel was neutralized with the same amount of 0.25N HCl and washed sufficiently with purified water to remove the unreacted BDDE. The washed gel was milled by using a plunger mill to a size of 20 $\mu$m and 200 $\mu$m. Hyaluronic acid content was determined through dry content and carbazole analysis from the gel content.

<Preparation of Composition for Freeze-Dried Formulation>

To 980 g of purified water, 5 g of crosslinked hyaluronic acid milled into a size of 20 $\mu$m, 10 g of low-molecular weight hyaluronic acid having a molecular weight of about 3,000 Da and 5 g of vitamin C were introduced. Then, the ingredients were mixed by using a homomixer. The resultant mixture was poured to a mold and frozen in a freeze-dryer at -20°C, followed by drying.

[Example 2]

To 980 g of purified water, 5 g of crosslinked hyaluronic acid milled into a size of 200 $\mu$m according to Example 1, 10 g of low-molecular weight hyaluronic acid having a molecular weight of about 3,000 Da and 5 g of vitamin C were introduced. Then, the ingredients were mixed by using a homomixer. The resultant mixture was poured to a mold and frozen in a freeze-dryer at -20°C, followed by drying.

[Comparative Example 1]

To 980 g of purified water, 15 g of low-molecular weight hyaluronic acid having a molecular weight of about 3,000 Da and 5 g of vitamin C were introduced. Then, the ingredients were mixed by using a homomixer. The resultant mixture was poured to a mold and frozen in a freeze-dryer at -20°C, followed by drying.

[Comparative Example 2]

To 980 g of purified water, 5 g of non-crosslinked hyaluronic acid having a molecular weight of about 1,000,000 Da, 10 g of low-molecular weight hyaluronic acid having a molecular weight of about 3,000 Da and 5 g of vitamin C were introduced. Then, the ingredients were mixed by using a homomixer. The resultant mixture was poured to a mold and frozen in a freeze-dryer at -20°C, followed by drying.

[Comparative Example 3]

To 980 g of purified water, 5 g of carboxymethyl, 10 g of low-molecular weight hyaluronic acid having a molecular weight of about 3,000 Da and 5 g of vitamin C were introduced. Then, the ingredients were mixed by using a homomixer. The resultant mixture was poured to a mold and frozen in a freeze-dryer at -20°C, followed by drying.

**Test Examples**

[Test Example 1- Property and Shape]

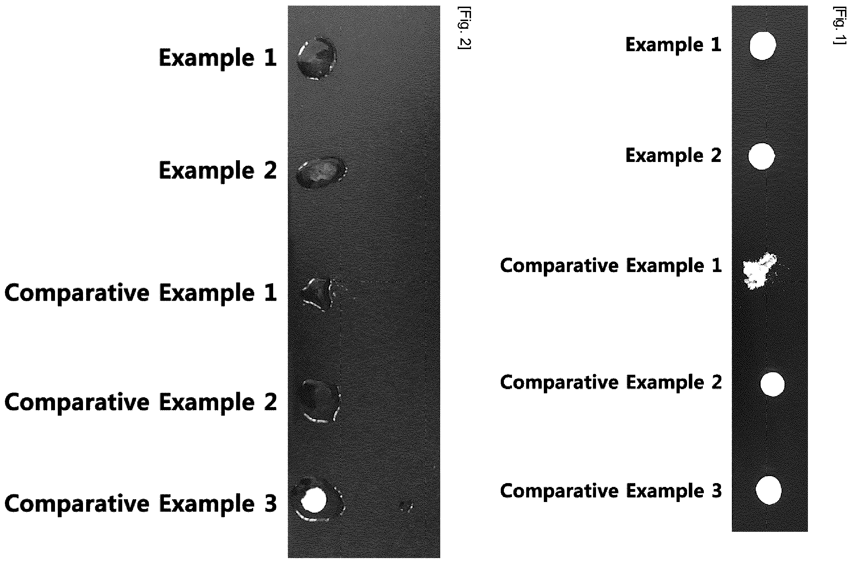

After determining the property and shape of each formulation after freeze-drying, the formulations according to Examples 1 and 2 and Comparative Example 2 have a fixed shape but the formulation according to Comparative Example 1 was crumbled, as shown in FIG. 1. Thus, it can be seen that it is not possible to retain a freeze-dried shape when using low-molecular weight hyaluronic acid alone.

[Test Example 2- Determination of Solubility]

In the above examples, 1 g of each of the mixtures was introduced to a mold and freeze-dried to obtain a freeze-dried formulation, and then 1 mL of purified water was poured to 20 mg of each freeze-dried formulation to determine the solubility. Referring to FIG. 2, the formulation according to Example 1 absorbs water and is converted into a gel-like shape. Also, in the case of Example 2, it is shown that the formulation absorbs water and is converted into a gel-like shape, but does not absorb water completely. It is thought that this is because small particles rapidly absorb water but large particles show a relatively low water absorption rate, due to the effect of surface area.

In the case of Comparative Examples 2, the formulation absorbs water and is converted into a slightly viscous solution state. In the case of Comparative Example 3, the formulation cannot absorb water and is not dissolved. Particularly, in the case of Comparative Example 3, the formulation is not dissolved, even when rubbing it with a hand, and carboxymethyl cellulose used for the formulation causes aggregation.

[Test Example 3- Comparison of Rheological Properties]

In the above examples, except Comparative Example 1 forming no formulation and Comparative Example 3 not dissolved in water, 1 g of each of the mixtures was introduced to a mold and freeze-dried to obtain a freeze-dried formulation, and then 20 mg of each freeze-dried formulation was dissolved in 1 mL of purified water. Then, the storage modulus (G') and loss modulus (G") were determined by using a rheometer (Kinexus Lab+ Malven), and the loss coefficient (tan$\delta$) was calculated based on them. The results are shown in the following Table 2.

As the storage modulus is increased, the formulation shows solid properties. As the loss coefficient is increased, the formulation shows liquid properties. In the case of Comparative Example 2, the formulation has a storage modulus of 0.58 $\pm$ 0.2 pa and a loss coefficient of 2.43 $\pm$ 0.48. Thus, it can be seen that the formulation according to Comparative Example 2 shows its unique liquid properties. On the contrary, in the case of Example 1, the formulation has a storage modulus of 7.06 $\pm$ 0.69 and a loss coefficient of 1.09 $\pm$ 0.07. Thus, it can be seen that the formulation according to Example 1 shows flowable weak gel properties. Example 2 has a storage modulus of 34.85 and a loss coefficient of 0.82. Thus, it can be seen that the formulation according to Example 2 shows hardly flowable and slightly hard gel properties.

[Table 2]

|  | Storage modulus (G', pa) | Loss modulus (G", pa) | Tan$\delta$ |
|---|---|---|---|
| Ex. 1 | 7.06 $\pm$ 0.69 | 7.7 $\pm$ 0.75 | 1.09 $\pm$ 0.07 |
| Ex. 2 | 34.85 | 28.63 | 0.82 |
| Comp. Ex. 2 | 0.58 $\pm$ 0.2 | 1.39 $\pm$ 0.45 | 2.43 $\pm$ 0.48 |

[Test Example 4- Evaluation of Feeling of Use]

Each of the formulations according to Examples 1 and 2 and Comparative Example 2 was dissolved in 1 mL of water, and then each of five subjects was allowed to apply each dissolved formulation to the face and to evaluate a feeling of moisturization and stickiness according to the following criteria. The results are shown in the following Table 3.
Stickiness: ◎ not sticky, ○ sticky, Δ very sticky
Feeling of Moisturization: ◎ excellent, ○ moderate, Δ poor
Dirt-rubbing: the cosmetic formulation causes rubbing-off like dirt.

[Table 3]

|  | Stickiness | Feeling of moisturization | Dirt-rubbing |
|---|---|---|---|
| Ex. 1 | ◎ | ◎ | No |
| Ex. 2 | ◎ | ○ | Yes |
| Comp. Ex. 2 | Δ | ○ | No |

After evaluating a feeling of use by the subjects, Examples 1 and 2 using crosslinked hyaluronic acid show no stickiness, but Comparative Example 2 shows slipperiness and stickiness unique to hyaluronic acid. In the case of Example 2, the crosslinked hyaluronic acid gel particles are excessively large so that they may be touched by fingers, and the formulation is not absorbed to the skin and causes dirt-rubbing.

[Test Example 5- Evaluation of Skin Moisturizing Effect]

To evaluate the moisturizing effect of each formulation, skin water content was determined for six 20-40 aged female and male subjects by using a corneometer (Amorepacific, Korea). The upper arm of each of subject was cleaned with a cleanser and dried for 10 minutes. Then, skin water content was determined.

A zone having a diameter of 2 cm was marked on the upper arm. Each of the samples according to Example 1 and Comparative Example 1 was obtained by introducing 1 g of each of the mixtures to a mold and freeze-drying it to obtain a freeze-dried formulation, and then dissolving 20 mg of each freeze-dried formulation in 1 mL of purified water. Then, the marked zone was treated with 0.2 mL of each sample. After the lapse of 1 hour, the water content at the treated site was determined by using a corneometer. The results are shown in the following Table 4.

After determining the skin water content, it is shown that the upper arm before the treatment shows a water content of $37.3 \pm 2.42\%$, Example 1 provides a water content of $52 \pm 3.69$, and Comparative Example 1 provides a water content of $44.8 \pm 1.83$. Thus, Example 1 shows the best feeling of moisturization with a statistic significance as verified by the t-test.

[Table 4]

| No. | Before treatment | Ex. 1 | Comp. Ex. 1 |
|---|---|---|---|
| 1 | 35 | 52 | 45 |
| 2 | 36 | 48 | 43 |
| 3 | 39 | 47 | 45 |
| 4 | 38 | 55 | 45 |
| 5 | 35 | 55 | 43 |
| 6 | 41 | 55 | 48 |
| Average | 37.3 | 52.0 | 44.8 |
| Deviation | 2.42 | 3.69 | 1.83 |

[Test Example 6- Data about Effect of Crosslinked HA/Low-Molecular Weight HA Ratio]

The effect of a crosslinked HA/low-molecular weight HA ratio was determined. The results are shown in the following Table 5. Each of the formulations according to Example 2 (crosslinked : low-molecular weight = 1 : 2), Example 3 (crosslinked : low-molecular weight = 1 : 1), Comparative Example 4 (crosslinked : low-molecular weight = 4 : 1) and Comparative Example 5 (crosslinked : low-molecular weight = 1 : 6) was evaluated in terms of a feeling of use and formulation stability.

In the case of Example 3 and Comparative Examples 4 and 5, each formulation was prepared by using the same crosslinked hyaluronic acid and low-molecular weight hyaluronic acid as used in Example 2, while the ratio between them was varied.

[Table 5]

| | Ex. 2 | Ex. 3 | Comp. Ex. 4 | Comp. Ex. 5 |
|---|---|---|---|---|
| Ratio | 1 : 2 | 1 : 1 | 4 : 1 | 1 : 6 |
| Feeling of use | Excellent | Excellent | Stiff, dirt-rubbing | Not formed |
| Formulation stability | Excellent | Excellent | Good shape but poor solubility | Not formed |

As can be seen from Table 5, Examples 2 and 3 show an excellent feeling of use and excellent formulation stability. On the contrary, Comparative Example 4 shows a stiff feeling of use and has poor solubility, and Comparative Example 5 which includes crosslinked HA providing physical properties at a low content (about 3 mg) cannot retain its shape after freeze-drying and the formulation itself cannot be formed.

Embodiment 1: A composition of freeze-dried formulation,
wherein the composition of freeze-dried formulation is mixed with a medium composition to be applied to a skin, and the composition of freeze-dried formulation includes freeze-dried hyaluronic acid,
wherein the freeze-dried hyaluronic acid includes crosslinked hyaluronic acid and low-molecular weight hyaluronic acid,
the freeze-dried hyaluronic acid is present in an amount of 0.5 dry wt% or more based on a total weight of the composition of freeze-dried formulation and the medium composition, when the composition of freeze-dried formulation is mixed with the medium composition to be applied to the skin, and
a weight ratio of the crosslinked hyaluronic acid to the low-molecular weight hyaluronic acid is 1:5 to 3:1.
Embodiment 2: The composition of freeze-dried formulation as defined in Embodiment 1, wherein the crosslinked hyaluronic acid is crosslinked with an epoxide-based crosslinking agent.
Embodiment 3: The composition of freeze-dried formulation as defined in Embodiment 1 or 2, wherein the crosslinked hyaluronic acid is crosslinked with 1,4-butanediol diglycidyl ether (BDDE), ethylene glycol diglycidyl ether (EGDGE), or 1-(2,3-epoxypropyl)-2,3-epoxycyclohexane.
Embodiment 4: The composition of freeze-dried formulation as defined in any one of Embodiments 1 to 3, wherein the crosslinked hyaluronic acid has a weight average molecular weight of 10,000,000 Da or more, and
the low-molecular weight hyaluronic acid has a weight average molecular weight of 5,000 Da or less.
Embodiment 5: The composition of freeze-dried formulation as defined in any one of Embodiments 1 to 4, wherein the crosslinked hyaluronic acid has a particle size less than 200 $\mu$m.
Embodiment 6: The composition of freeze-dried formulation as defined in any one of Embodiments 1 to 5, which further includes, as an active ingredient, at least one selected from the group consisting of water-soluble vitamins, oil-soluble vitamins, vitamin derivatives, polymeric peptides, polysaccharides, spingolipids and seaweed extract.
Embodiment 7: The hyaluronic acid composition for external use on skin as defined in any one of Embodiments 1 to 6, which is a cosmetic composition.
Embodiment 8: A cosmetic kit for external use on skin which includes: a first agent which is the composition of freeze-dried formulation as defined in any one of Embodiments 1 to 7; and a second agent which is a medium composition mixed with the first agent to be applied to the skin, wherein the first agent and the second agent are mixed with each other to form a gel composition for external use on skin, and the gel composition for external use on skin includes freeze-dried hyaluronic acid in an amount of 0.5 dry wt% or more based on the total weight of the gel composition for external use on skin.
Embodiment 9: The cosmetic kit for external use on skin as defined in Embodiment 8, wherein the second agent is at least one selected from the group consisting of ampoule, cream, skin softener, skin astringent, skin nutrient, nutrient cream, massage cream, essence, eye cream, eye essence, cleansing cream and cleansing water.
Embodiment 10: The cosmetic kit for external use on skin as define din Embodiments 8 or 9, wherein the gel composition for external use on skin has a tan$\delta$ value of 0.5-1.5, as determined according to the following Formula 1:

$$[\text{Formula 1}]$$

$$\text{Tan}\delta = G''/G'$$

wherein G" is the loss modulus of the gel composition for external use on skin, and G' is the storage modulus of the gel composition for external use on skin.
Embodiment 11: A non-therapeutic use of the composition of freeze-dried formulation according to any one of Embodiments 1-7 for skin moisturization.

**Claims**

1. A composition of freeze-dried formulation,
   wherein the composition of freeze-dried formulation is mixed with a medium composition to be applied to a skin, and
   the composition of freeze-dried formulation comprises freeze-dried hyaluronic acid,
   wherein the freeze-dried hyaluronic acid comprises crosslinked hyaluronic acid and low-molecular weight hyaluronic acid,
   the freeze-dried hyaluronic acid is present in an amount of 0.5 dry wt% or more based on a total weight of the composition of freeze-dried formulation and the medium composition, when the composition of freeze-dried formulation is mixed with the medium composition to be applied to the skin, and
   a weight ratio of the crosslinked hyaluronic acid to the low-molecular weight hyaluronic acid is 1:5 to 3:1.

2. The composition of freeze-dried formulation according to claim 1, wherein the crosslinked hyaluronic acid is crosslinked with an epoxide-based crosslinking agent.

3. The composition of freeze-dried formulation according to claims 1 or 2, wherein the crosslinked hyaluronic acid is crosslinked with at least one selected from the group consisting of 1,4-butanediol diglycidyl ether (BDDE), ethylene glycol diglycidyl ether (EGDGE), 1-(2,3-epoxypropyl)-2,3-epoxycyclohexane, 1,2-ethanediol diglycidyl ether, 1,6-hexanediol diglycidyl ether, propylene glycol diglycidyl ether, polypropylene glycol diglycidyl ether, and diglycerol polyglycidyl ether.

4. The composition of freeze-dried formulation according to any one of claims 1-3, wherein the crosslinked hyaluronic acid has a weight average molecular weight of 10,000,000 Da or more, and
the low-molecular weight hyaluronic acid has a weight average molecular weight of 5,000 Da or less.

5. The composition of freeze-dried formulation according to any one of claims 1-4, wherein the crosslinked hyaluronic acid has a particle size less than 200 $\mu$m.

6. The composition of freeze-dried formulation according to any one of claim 1-5, wherein the composition of freeze-dried formulation further comprises, as an active ingredient, at least one selected from the group consisting of water-soluble vitamins, oil-soluble vitamins, vitamin derivatives, polymeric peptides, polysaccharides, spingolipids and seaweed extract.

7. The composition of freeze-dried formulation according to any one of claims 1-6, wherein the composition of freeze-dried formulation is a cosmetic composition.

8. A cosmetic kit for external use on skin comprising:

a first agent which is the composition of freeze-dried formulation as defined in any one of claims 1 to 7; and
a second agent which is a medium composition mixed with the first agent to be applied to the skin,
wherein the first agent and the second agent are mixed with each other to form a gel composition for external use on skin, and
the gel composition for external use on skin comprises freeze-dried hyaluronic acid in an amount of 0.5 dry wt% or more based on the total weight of the gel composition for external use on skin.

9. The cosmetic kit for external use on skin according to claim 8, wherein the second agent is at least one selected from the group consisting of ampoule, cream, skin softener, skin astringent, skin nutrient, nutrient cream, massage cream, essence, eye cream, eye essence, cleansing cream and cleansing water.

10. The cosmetic kit for external use on skin according to claims 8 or 9, wherein the gel composition for external use on skin has a tan$\delta$ value of 0.5-1.5, as determined according to the following Formula 1:

[Formula 1]

$$\text{Tan}\delta = G''/G'$$

wherein G" is the loss modulus of the gel composition for external use on skin, and G' is the storage modulus of the gel composition for external use on skin.

11. A non-therapeutic use of the composition of freeze-dried formulation according to any one of claims 1-7.

[Fig. 1]

[Fig. 2]

[Fig. 3]

Content of Skin moisture (%)

Europäisches
Patentamt

European
Patent Office

Office européen
des brevets

## EUROPEAN SEARCH REPORT

Application Number

EP 20 18 8071

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 924 606 A1 (THOREL JEAN NOEL [FR]) 12 June 2009 (2009-06-12) | 1-11 | INV. A61K8/73 A61Q19/00 |
| Y | * page 6; claims 1-12; examples 1, 2, 3 * | 1-11 | |
| X | YASUHIRO MATSUMOTO ET AL: "Development of a Wound Dressing Composed of Hyaluronic Acid Sponge Containing Arginine and Epidermal Growth Factor", JOURNAL OF BIOMATERIALS SCIENCE. POLYMER EDITION., vol. 21, no. 6-7, 2 January 2010 (2010-01-02), pages 715-726, XP055762064, NL ISSN: 0920-5063, DOI: 10.1163/156856209X435844 * page 717 * | 1-11 | |
| X | US 2011/003769 A1 (KIM KI HO [KR] ET AL) 6 January 2011 (2011-01-06) * examples 1, 2, 3; tables 1, 2, 3, 6, 7 * | 1-11 | |
| Y | CN 106 726 709 B (HUNAN YUJIA COSMETIC MFG CO LTD) 1 March 2019 (2019-03-01) * the whole document * | 1-11 | TECHNICAL FIELDS SEARCHED (IPC) A61K A61Q |

The present search report has been drawn up for all claims

| Place of search | Date of completion of the search | Examiner |
|---|---|---|
| Munich | 22 December 2020 | Szarek, Sophie |

EPO FORM 1503 03.82 (P04C01)

## ANNEX TO THE EUROPEAN SEARCH REPORT
## ON EUROPEAN PATENT APPLICATION NO.

EP 20 18 8071

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2020

| Patent document cited in search report | | Publication date | Patent family member(s) | | Publication date |
|---|---|---|---|---|---|
| FR 2924606 | A1 | 12-06-2009 | NONE | | |
| US 2011003769 | A1 | 06-01-2011 | JP | 5517955 B2 | 11-06-2014 |
| | | | JP | 2011513481 A | 28-04-2011 |
| | | | KR | 20090098083 A | 17-09-2009 |
| | | | US | 2011003769 A1 | 06-01-2011 |
| | | | WO | 2009113820 A1 | 17-09-2009 |
| CN 106726709 | B | 01-03-2019 | NONE | | |

EPO FORM P0459

For more details about this annex : see Official Journal of the European Patent Office, No. 12/82